# EUROPÄISCHE PATENTSCHRIFT

(11) **EP 0 776 629 B1**
(45) Veröffentlichungstag und Bekanntmachung des Hinweises auf die Patenterteilung: **23.06.2004**
(21) Anmeldenummer: 96117533.8
(22) Anmeldetag: 31.10.1996
(51) Int. Cl.: A61B 5/0408

(54) **Elektrodenapplikationsvorrichtung**
System for applying electrodes
Système d'application pour électrode

(30) Priorität: 08.11.1995 DE 19541627
(43) Veröffentlichungstag der Anmeldung: 04.06.1997
(73) Patentinhaber: GE Medical Systems Information Technologies GmbH, 79111 Freiburg i. Br. (DE)
(72) Erfinder: Huber, Peter, 79331 Teningen (DE)
(74) Vertreter: Müller, Frithjof E., Dipl.-Ing.

(56) Entgegenhaltungen:
- EP-A- 0 103 793
- WO-A-93/16633
- DE-A- 1 514 345

## Beschreibung

Die Erfindung betrifft eine Elektrodenapplikationsvorrichtung für die EKG-Diagnostik mit einer über eine Versorgungsleitung an eine Unterdruckpumpe anschließbaren Unterdruck-Verteilereinrichtung, über die eine Mehrzahl von Saugelektroden mittels jeweils zugeordneter Unterdruckleitung an eine entsprechende Anzahl von individuell zugeordneten Ventilen angeschlossen ist, die eingangsseitig über die Unterdruck-Verteilereinrichtung mit Unterdruck beaufschlagbar sind, wobei die Unterdruck-Verteilereinrichtung einen Versorgungskanal und einen Belüfter aufweist und sich an den Versorgungskanal ein Verteilerkanal anschließt, an den die Ventile angeschlossen sind, wobei der Verteilerkanal (20) über einen belüftbaren Unterdruckregler (4, 14) an den Versorgungskanal (3) angeschlossen ist.

In der EP-A-0 103 793 ist eine Saugeinrichtung zur Applikation von Saugelektroden und zur Saugmassage beschrieben, bei der Belüftungsventile vorgesehen sind, mit deren Hilfe durch einströmende Luft ein weiteres Ansteigen eines Unterdruckes in einer Saugleitung verhindert werden soll. Ein Ventil wird so weit und so lange geöffnet, bis die hierdurch einströmende Luft ein weiteres Ansteigen des Unterdrucks in der Saugleitung unterbindet.

Für eine zuverlässige EKG-Diagnostik ist die korrekte ungestörte Abnahme der Herzaktionsspannungen von der Körperoberfläche eine der wichtigsten Vorrausetzungen für ein aussagefähiges Elektrokardiogramm. Besonders bei der EKG-Aufzeichnung während der Ergometrie ist eine gute Haftung der Elektroden auf der Haut von entscheidender Bedeutung.

Für die Abnahme der Herzaktionsspannungen sind Saugelektrodensysteme bekannt, bei denen die Elektroden, beispielsweise Silber-Silberchlorid-Elektroden, von Saugnäpfen aus flexiblem Kunststoffmaterial umgeben sind, die über flexible Unterdruckleitungen an regelbare, individuell zugeordnete Ventile innerhalb einer blockartigen Unterdruck-Verteilervorrichtung angeschlossen sind, die ihrerseits über einen Versorgungskanal an eine durch einen Drucksensor gesteuerte elektrisch betriebene Saugpumpe angeschlossen ist.

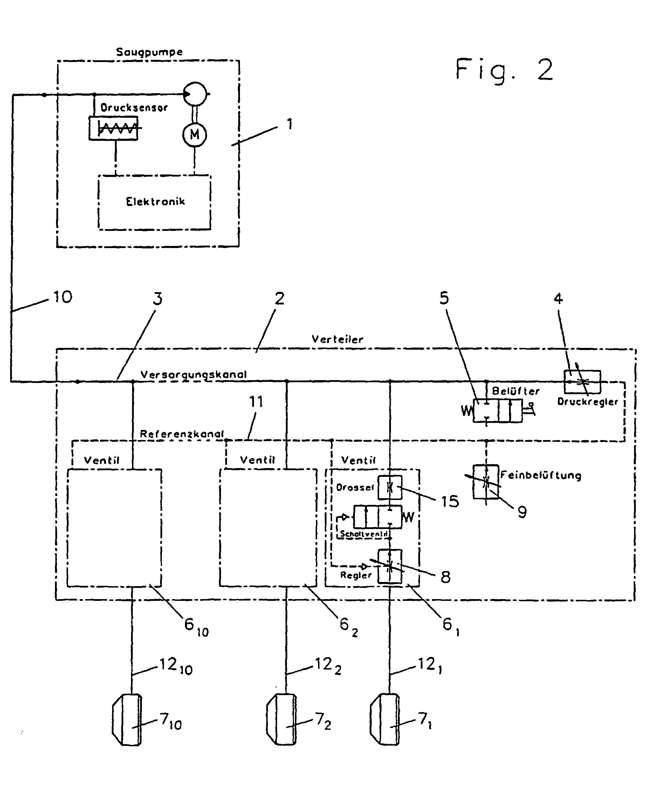

Die **Fig. 2** der beigefügten Zeichnungen veranschaulicht den prinzipiellen Aufbau einer bekannten Elektrodenapplikationsvorrichtung mit der erwähnten, mittels Drucksensor über eine empfindliche Steuerelektronik gesteuerten Saugpumpe 1, die über eine Unterdruck-Versorgungsleitung 10 unmittelbar an einen Versorgungskanal 3 innerhalb einer in der Regel als blockartige Baueinheit realisierten Verteilereinrichtung 2 angeschlossen ist. Der Versorgungskanal 3 ist einerseits an einen jeweils ersten eingangsseitigen Anschluß einer Mehrzahl von Ventilen 6₁, 6₂, ... über je einen Durchflußbegrenzer 15 direkt angeschlossen. Über einen jeweils zweiten eingangsseitigen Anschluß ist ein jeweiliger individuell einstellbarer Druckregler 8 innerhalb jedes der Ventile 6₁, 6₂, ... an einen Druck-Referenzkanal 11 angeschlossen, der seinerseits über einen Druckregler 4 mit dem Versorgungskanal 3 verbunden ist. Ausgangsseitig sind die Ventile 6₁, 6₂, ... über die erwähnten flexiblen Unterdruckleitungen 12₁, 12₂, ... an die nicht in Einzelheiten dargestellten Saugnäpfe der Elektroden 7₁, 7₂, ... angeschlossen. Vom Druck-Referenzkanal 11 aus wird den jedem Ventil zugeordneten Reglern 8 über den Druckregler 4 ein bestimmter Druck-Referenzwert vorgegeben, um das jeweilige Ventil ein- bzw. auszuschalten, um so einen möglichst konstanten Unterdruck an der Applikationsstelle der jeweiligen Elektroden sicherzustellen. Um eine Grenze für den unteren Druckwert innerhalb des Druck-Referenzkanals 11 zu erhalten, ist zusätzlich eine Feinbelüftung 9 beispielsweise mit Feinverstellung über eine Madenschraube vorgesehen. Der Versorgungskanal 3 und damit die Verteilereinrichtung 2 lassen sich über ein Belüfterventil 5 auf Umgebungsdruck belüften, so daß die applizierten Elektroden 7₁, 7₂, ... problemlos abgenommen werden können.

Die bekannte Elektrodenapplikationsvorrichtung, wie sie beispielhaft in Fig. 2 gezeigt ist, arbeitet zufriedenstellend und ermöglicht eine einfache und zuverlässige Elektrodenapplikation sowohl in der EKG-Diagnostik als auch ggfs, für andere Anwendungsbereiche, bei denen mit Unterdruck-Saugelektroden gearbeitet wird, etwa im Bereich der transkutanen pO/pCO₂-Diagnostik oder bei der Applikation von Defibrillatorelektroden sowie von Elektroden für die Physiotherapie.

Gelegentliche Probleme ergeben sich bei der bekannten Elektrodenapplikationsvorrichtung durch die durch den Druckregler 4 über die individuell zugeordneten Regler 8 einstellbaren Ventile 6₁, 6₂, insbesondere bei glatter, trockener Haut des Probanden oder bei eng beieinanderstehender Elektrodenapplikation. Dies insbesondere deshalb, weil die jeweilige Regelung nur in einer Richtung, zum Beispiel nur in Richtung auf stärkeren Unterdruck erfolgt. Es läßt sich dann beim Abschalten und Belüften kein einwandfreier Abwurf aller Elektroden erreichen. Denkbar wäre der Einbau von zwei Reglern innerhalb jedes Ventils oder jeweils eines Reglers mit automatischer Belüftung. Abgesehen von einer baulichen Vergrößerung würde dies jedoch die Verteilereinrichtung 2 erheblich verteuern.

Der Erfindung liegt damit die Aufgabe zugrunde, eine Elektrodenapplikationsvorrichtung der eingangs genannten Gattung so zu verbessern, daß auch bei problematischen Applikationsfällen ein einheitliches Abfallen der Elektroden gewährleistet ist. Gleichzeitig soll eine konstruktive Vereinfachung und damit kostengünstigere Herstellung der genannten Applikationsvorrichtung erreicht werden.

Diese Aufgabe wird bei einer Elektrodenapplikationsvorrichtung der eingangs genannten Art erfindungsgemäß durch die im kennzeichnenden Teil des Patentanspruchs 1 angegebenen Merkmale gelöst.

Vorteilhafte Ergänzungen und Ausgestaltungen des Erfindungsgedankens sind in abhängigen Patentansprüchen gekennzeichnet.

Im Gegensatz zur bekannten Verteilereinrichtung, wie sie in Fig. 2 gezeigt ist, sieht die Erfindung vor, die einzelnen Ventile eingangsseitig direkt an einen gemeinsamen Verteilerkanal anzuschließen, dessen Druck über einen gemeinsamen belüftbaren Druckregler bestimmt wird, der die Verbindung zum Versorgungskanal innerhalb derverteilereinrichtung herstellt. Auf diese Weise wird der Aufbau der Ventile nicht nur erheblich vereinfacht, da der individuell zugeordnete Regler entfällt. Die Ventile können auch in erheblich verkleinerter Bauweise verwirklicht werden, weil jeweils nur ein eingangsseitiger Unterdruck-Anschlußstutzen benötigt wird.

Die Erfindung und vorteilhafte Einzelheiten sowie eine Ausführungsform der Erfindung werden nachfolgend unter Bezug auf die Zeichnung erläutert. Es zeigen:
**Fig. 1** die Prinzipdarstellung und den Anordnungsaufbau einer Elektrodenapplikationsvorrichtung mit erfindungsgemäßen Merkmalen, und
**Fig. 2** den bereits erläuterten Aufbau einer Elektrodenapplikationsvorrichtung gemäß dem Stand der Technik.

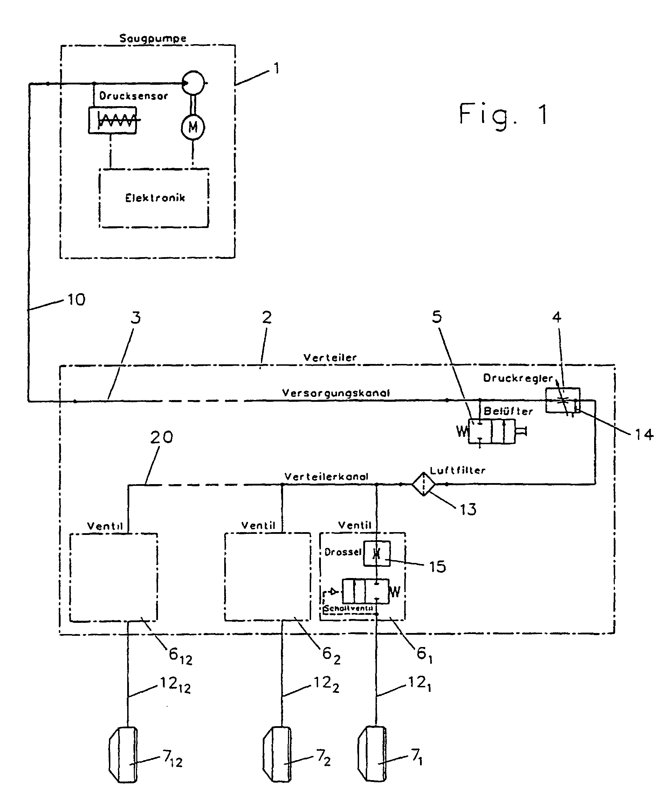

Einander entsprechende und anhand der Fig. 2 bereits erläuterte Baugruppen und Teile sind in Fig. 1 mit dem jeweils gleichen Bezugshinweis bezeichnet.

Wie die Fig. 1 in Vergleich zu Fig. 2 augenfällig erkennen läßt, ist der zwischen dem Versorgungskanal 3 und einem Verteilerkanal 20 angeordnete Druckregler 4 zur verstellbaren Vorgabe eines Soll-Unterdrucks mit einer Belüftungseinrichtung 14 versehen. Der Druckregler 4 wird über ein zweites, von derselben Membran gesteuertes Ventil belüftet, dessen Einzelheiten im vorliegenden Zusammenhang nicht von Interesse sind.

Vom Verteilerkanal 20 aus führen Stichverbindungen zu den einzigen eingangsseitigen Unterdruckanschlüssen der Ventile 6₁, 6₂,.... Im Verteilerkanal und vor den Abzweigungen zu den einzelnen Ventilen kann ein Luftfilter 13 vorgesehen sein. Weiterhin kann in jedem Stichkanal zu den Ventilen eine durchflußbegrenzende Drossel 15 vorgesehen sein.

Über den Verteilerkanal 20 werden die Saugnäpfe der Elektroden 7₁, 7₂, ... mit dem geregelten Unterdruck versorgt. Ersichtlicherweise entfallen damit die beim Stand der Technik nach Fig. 2 vorhandenen Regler 8 in den einzelnen Ventilblöcken bzw. Ventilen 6₁, 6₂,.... Außer einem geringeren Herstellungs- und Montageaufwand bei der Fertigung reduziert sich durch die erfindungsgemäße Vereinfachung auch die Möglichkeit von undichten Stellen.

Weiterhin ist erkennbar, daß durch den Wegfall des Referenzdrucks (Referenzkanal 11 in Fig. 2) auch der zweite Unterdruck-Anschlußstutzen an den einzelnen Ventilblöcken 6₁, 6₂, ... und natürlich der Referenzkanal selbst entfällt. Wird bei angelegten Elektroden 7₁, 7₂, ... der Unterdruck am zentralen, belüftenden Druckregler 4 reduziert, so stellt sich sofort der neue Unterdruck an den Elektroden ein. Dies ist ein wesentlicher Vorteil gegenüber der bisher bekannten Lösung, bei der keine Belüftung durch den Regler 4 möglich war.

Durch die neue erfindungsgemäße Gestaltung, insbesondere des Verteilerblocks 2, wird das Abfallen der Elektroden 7₁, 7₂, ... nach Übergang in die "Aus"-Stellung unabhängig vom vorherigen Unterdruck sicher ermöglicht. Ein eventuell verbleibender geringer Unterdruck der notwendig sein kann, um das der jeweiligen Elektrode 7₁, 7₂, ... vorgeschaltete Ventil 6₁, 6₂, ... zu öffnen bzw. offen zu halten, reicht nicht aus, um die Elektrode am Applikationsort zu halten. Ein sicheres Abfallen der Elektroden ist damit gewährleistet. Für das die EKG-Diagnostik begleitende und überwachende medizinische Personal ergibt sich damit bei der Anwendung eine zusätzliche hilfreiche Vereinfachung.

## Patentansprüche

1. Elektrodenapplikationsvorrichtung für die EKG-Diagnostik, mit einer über eine Versorgungsleitung (10) an eine Unterdruckpumpe (1) anschließbaren Unterdruck-Verteilereinrichtung (2), über die eine Mehrzahl von Saugelektroden (7₁, 7₂, ...) mittels jeweils zugeordneter Unterdruckleitung (12₁, 12₂, ...) an eine entsprechende Anzahl von individuell zugeordneten Ventilen (6₁, 6₂, ...) angeschlossen ist, die eingangsseitig über die Unterdruck-Verteilereinrichtung (2) mit Unterdruck beaufschlagbar sind, wobei die Unterdruck-Verteilereinrichtung (2) einen Versorgungskanal (3) und einen Belüfter (5) aufweist und sich an den Versorgungskanal (3) ein Verteilerkanal (20) anschließt, an den die Ventile (6₁, 6₂, ...) angeschlossen sind, wobei der Verteilerkanal (20) über einen zur verstellbaren Vorgabe eines Soll-Unterdrucks mit einer Belüftungseinrichtung (14) versehenen Druckregler (4) an den Versorgungskanal (3) angeschlossen ist, und dass der Versorgungskanal (3) mit einem Belüfterventil (5) verbunden ist, über das der Versorgungskanal und damit die Unterdruck-Verteilereinrichtung (2) auf Umgebungsdruck belüftbar sind.

2. Elektrodenapplikationsvorrichtung nach Anspruch 1, **dadurch gekennzeichnet, dass** die Ventile (6₁, 6₂, ...) nur einen eingangsseitigen Unterdruck-Anschluss aufweisen.

3. Elektrodenapplikationsvorrichtung nach Anspruch 1 oder 2, **dadurch gekeennzeichnet, dass** im Verteilerkanal (20) bzw. im Versorgungskanal (3) innerhalb der Unterdruck-Verteilereinrichtung (2) ein Luftfilter (13) vorgesehen ist.

4. Elektrodenapplikationsvorrichtung nach einem der vorstehenden Ansprüche 1 bis 3, **dadurch gekennzeichnet, dass** in den vom Verteilerkanal (20) zu den eingangsseitigen Ventilanschlüssen führenden Anschlusskanälen je eine Strömungsdrossel (15) angeordnet ist.

## Claims

1. A system for applying, electrodes for use in ECG diagnostics, having a vacuum distribution arrangement (2) which is capable of being connected to a vacuum pump (1) via a supply circuit (10) and is the means by which a plurality of suction electrodes (7₁, 7₂, ...) is connected by respectively associated vacuum tubing (12₁, 12₂, ...) to a corresponding number of individually associated valves (6₁, 6₂, ...) which, on their input side, are capable of being impinged with sub-atmospheric pressure via the vacuum distribution arrangement (2), whereby the vacuum distribution arrangement (2) comprises a supply channel (3) and a ventilator (5), with the supply channel (3) being linked to a distribution channel (20) to which are connected the valves (6₁, 6₂, ...),
whereby the distribution channel (20) is connected to the supply channel (3) by means of a pressure regulator (4) that is provided with a ventilating device (14) for the adjustable setting of a required sub-atmospheric pressure, and that the supply channel (3) is connected to a ventilator valve (5) by means of which the supply channel and thereby the vacuum distribution arrangement (2) are capable of being ventilated to the pressure of the surrounding atmosphere.

2. A system for applying electrodes according to claim 1, **characterised in that** the valves (6₁, 6₂, ...) comprise, on their input side, only one vacuum connection.

3. A system for applying electrodes according to claim 1 or 2, **characterised in that**, within the vacuum distribution arrangement (2), an air filter (13) is provided in the distribution channel (20) or, as the case may be, in the supply channel (3).

4. A system for applying electrodes according to one of the preceding claims 1 to 3, **characterised in that**, arranged in the connecting channels leading from the distribution channel (20) to the connections on the input side of the valves, there is, in each case, a flow control throttle (15).

## Revendications

1. Dispositif d'application d'électrodes pour le diagnostic ECG, avec un dispositif de répartition de dépression (2) qui, par l'intermédiaire d'une conduite d'alimentation (10), peut être relié à une pompe à vide (1) et par l'intermédiaire duquel une pluralité d'électrodes-ventouses (7₁, 7₂...) est reliée, au moyen d'une conduite de dépression respective associée (12₁, 12₂...), à un nombre correspondant de valves associées individuellement (6₁, 6₂...) qui, côté entrée, peuvent être soumises à une dépression par l'intermédiaire du dispositif de répartition de dépression (2), le dispositif de répartition de dépression (2) comportant un canal d'alimentation (3) et un aérateur (5), et un canal de répartition (20), auquel sont reliées les valves (6₁, 6₂...), se raccordant au canal d'alimentation (3), le canal de répartition (20) étant relié au. canal d'alimentation (3) par l'intermédiaire d'un régulateur de pression (4) muni d'un dispositif d'aération (14) pour la préaffectation réglable d'une dépression de consigne, et le canal d'alimentation (3) étant relié à une- valve d'aération (5) par l'intermédiaire de laquelle le canal d'alimentation et donc le dispositif de répartition de dépression (2) peuvent être ventilés à la pression ambiante.

2. Dispositif d'application d'électrodes selon la revendication 1, **caractérisé en ce que** les valves (6₁, 6₂...) ne comportent qu'une connexion de dépression côté entrée.

3. Dispositif d'application d'électrodes selon la revendication 1 ou 2, **caractérisé en ce qu'**un filtre à air (13) est prévu sur le canal de répartition (20), respectivement sur le canal d'alimentation (3) à l'intérieur du dispositif de répartition de dépression (2).

4. Dispositif d'application d'électrodes selon l'une des revendications précédentes 1 à 3, **caractérisé en ce que** sur chacun des canaux de liaison menant du canal de répartition (20) aux connexions de valves côté entrée est prévu un moyen d'étranglement d'écoulement (15).
